## Europäisches Patentamt

## European Patent Office

## Office européen des brevets

(11) Veröffentlichungsnummer: **0 012 800**
**B1**

(12) # EUROPÄISCHE PATENTSCHRIFT

(45) Veröffentlichungstag der Patentschrift:
**16.09.81**

(51) Int. Cl.³: **C 07 C 49/12**, C 07 C 45/33

(21) Anmeldenummer: **79103833.4**

(22) Anmeldetag: **08.10.79**

(54) Verfahren zur Herstellung von Butan-2,3-dion und Aceton aus Isobutanal.

(30) Priorität: **06.12.78 DE 2852716**

(43) Veröffentlichungstag der Anmeldung:
**09.07.80 Patentblatt 80/14**

(45) Bekanntmachung des Hinweises auf die Patenterteilung:
**16.09.81 Patentblatt 81/37**

(84) Benannte Vertragsstaaten:
**BE CH FR GB IT NL**

(56) Entgegenhaltungen:
**DE-B-1 232 126**
**DE-C-1 162 345**
**US-A-2 686 813**

**CHEMICAL ABSTRACTS,**
**vol. 82, 1975**
**Columbus, Ohio, USA**
**124 770p**

(73) Patentinhaber: **CHEMISCHE WERKE HÜLS AG,**
**Postfach 1320, D-4370 Marl 1 (DE)**

(72) Erfinder: **Scharf, Helmut, Dr., Schetterstrasse 82,**
**D-4235 Schermbeck (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG.

Verfahren zur Herstellung von Butan-2.3-dion und Aceton aus Isobutanal

Butan-2: 3-dion (Diacetyl) ist ein in der Natur weit verbreiteter Aromastoff und stellt ein wertvolles Ausgangsmaterial für chemische Synthesen dar. Es sind daher für seine Herstellung eine Reihe von Verfahren sowohl für die Flüssigphase als auch für die Gasphase entwickelt worden (siehe z. B. Beilstein, Band 1, I. – IV. Ergänzung). Auch die Verwendung von Isobutanal als Ausgangsstoff für die Synthese von Butan-2.3-dion ist bereits beschrieben worden. So wird zum Beispiel in der DE-PS 11 62 345 Isobutanal in der Flüssigphase mit Kupfer(II)-chlorid oder Eisen(III)-chlorid oxidiert. Bei diesem Verfahren werden jedoch für die Oxidation sehr grosse (überstöchiometrische) Mengen an Salzen benötigt und zusätzlich treten grosse Korresionsprobleme auf. In der JP-PS 766 233 wird ein Verfahren vorgeschlagen, bei dem Isobutanal in der Gasphase bei 350°C an einem Katalysator umgesetzt wird, der aus den Oxiden von Molybdän, Vanadin und Antimon besteht. Bei diesem Verfahren entstehen neben Butan-2. 3-dion grössere Mengen an Methacrolein, Aceton, Essigsäure und Acetaldehyd. Die Bildung von mehreren Koppelprodukten macht die Aufarbeitung des Reaktionsproduktes schwierig und ist auch aus markttechnischen Gründen unerwünscht. Hinzu kommt, dass die entstehende Essigsäure Korrosionsprobleme aufwirft. Die bei diesem Verfahren erforderlichen hohen Reaktionstemperaturen erfordern ausserdem Vielrohrreaktoren, die mit Hilfe von Salzschmelzen thermostatisiert werden müssen, so dass das Verfahren einen hohen Investitionsaufwand erfordert und ausserdem schwierig zu betreiben ist. In der japanischen Patentanmeldung 6201/68 wird ein Verfahren mit einem Katalysator aus Molybdänoxid in Kombination mit den Oxiden von Eisen, Wismut, Antimon, Tellur oder Kobalt vorgeschlagen. Bei diesem Verfahren beträgt jedoch die Ausbeute an Butan-2.3-dion und Aceton maximal 64 Mol-%. Über die restlichen 36 Mol-% werden keine Angaben gemacht, jedoch ist zu vermuten, dass auch bei diesem Verfahren Methacrolein und Essigsäure als weitere Nebenprodukte auftreten.

Aufgabe der vorliegenden Erfindung ist es, ein Verfahren zur Herstellung von Butan-2,3-dion durch katalytische Oxidation von Isobutanal in der Gasphase zu entwickeln, bei dem neben Butan-2.3-dion hauptsächlich nur Aceton entsteht, bei dem die Summe der Selektivitäten von Butan-2.3-dion und Aceton möglichst hoch ist und das auf Salzschmelzenreaktoren verzichten kann.

Diese Aufgabe wird erfindungsgemäss entsprechend den Angaben des Patentanspruchs gelöst.

Das erfindungsgemässe Verfahren hat die Vorteile, dass als Koppelprodukt zum Butan-2.3-dion praktisch nur Aceton entsteht, die Ausnutzung des Einsatzstoffes sehr hoch ist und die Reaktion bei relativ niedrigen Temperaturen in mit Siedewasser thermostatisierten Vielrohrreaktoren durchgeführt werden kann, so dass das Verfahren trotz der relativ niedrigen Selektivität für Butan-2.3-dion wirtschaftlicher arbeitet.

Die erfindungsgemässen Katalysatoren lassen sich als Voll- oder Trägerkatalysatoren nach den üblichen Methoden herstellen. Bevorzugt werden Vollkatalysatoren, da einige in der Technik übliche Träger einen selektivitätsmindernden Einfluss auf die Reaktion ausüben. Wegen der Vielzahl der möglichen Träger muss im Einzelfall der vorgesehene Träger unter Reaktionsbedingungen auf seine selektivitätsmindernden Eigenschaften geprüft werden. Der Manganoxidgehalt des fertigen Katalysators beträgt 0,1 bis 10 Gewichtsprozent. Unter 0,1 Gewichtsprozent wird der Umsatz für technische Zwecke zu niedrig und Konzentrationen über 10 Gewichtsprozent bringen keine zusätzlichen Vorteile mehr. Ausserdem nimmt bei höheren Konzentrationen der Anteil an Totalverbrennung des Aldehyds zu. Bevorzugt wird ein Gehalt an Mangan in Form des Oxids von 1 bis 3 Gewichtsprozent. Der Zinkoxidgehalt des fertigen Katalysators beträgt vorzugsweise 1 bis 80 Gewichtsprozent, der Titandioxidgehalt vorzugsweise 1 bis 60 Gewichtsprozent. Niedrigere Konzentrationen zeigen keinen deutlichen Einfluss mehr. Die obere Grenze des Zink- und/oder Titangehaltes des fertigen Katalysators richtet sich nach dem Mangangehalt und der gegebenenfalls verwendeten Menge an Trägern und wird durch die Bedingung festgelegt, dass sich die prozentuale Menge der Metalloxide und die prozentuale Menge des gegebenenfalls verwendeten Trägers zu 100% ergänzen müssen.

Bei Trägerkatalysatoren beträgt der Zinkoxid- und/oder Titandioxidgehalt in der Regel 1 bis 20 Gewichtsprozent. besonders bevorzugt werden Zinkoxid- und/oder Titandioxidgehalte von 2 bis 5 Gewichtsprozent. Bei den besonders bevorzugten Vollkatalysatoren dient das Zink- und/oder Titanoxid gleichzeitig als Träger für das Manganoxid. Bevorzugt werden Katalysatoren, bei denen das Manganoxid jeweils nur mit Zinkoxid oder Titanoxid kombiniert ist, da die Dreierkombination der Oxide von Mangan, Zink und Titan gegenüber der Kombination des Manganoxids mit nur einem anderen Metalloxid keine Vorteile bringt. Bei der Verwendung von Vollkatalysatoren hat es sich als vorteilhaft erwiesen, dem Zink- und/oder Titanoxid 2 bis 20 Gewichtsprozent Kolloidgraphit, bezogen auf die Zinkoxid- und/oder Titandioxid-Menge, hinzuzufügen. Bevorzugt werden Graphitgehalte von 2 bis 5 Gewichtsprozent, bezogen auf die Zinkoxid- und/oder Titandioxid-Menge, da grössere Mengen an Graphit keine weiteren Vorteile mehr bringen. Für das Zink- und das Titanoxid und den Kolloidgraphit werden handelsübliche Qualitäten verwendet.

Die Herstellung des Katalysators erfolgt nach den in der Technik üblichen Methoden. So kann zum Beispiel ein Gemisch aus Manganoxid, Zinkoxid und Graphit durch Tablettieren oder Extrudieren in eine geeignete Form gebracht werden. Auch ist es zum Beispiel möglich, ein Gemisch aus Zinkoxid und Graphit mit einer wässrigen Mangansalzlösung, zum Beispiel einer wässrigen Lösung von Mangannitrat zu imprägnieren, anschliessend zu trocknen und die Komponente in bekannter Weise durch Erhitzen in Gegenwart von Sauerstoff in die Oxidform umzuwandeln und in eine geeignete Form zu bringen. Es ist ebenfalls möglich, zunächst Formlinge aus Zinkoxid und Graphit herzustellen, diese mit einer wässrigen Mangansalzlösung oder einem organischen Mangankomplex zu imprägnieren, zu trocknen und anschliessend zu kalzinieren. Des weiteren kann der Katalysator durch Eindampfen einer Suspension von Kolloidgraphit in der entsprechenden wässrigen Metallsalzlösung und Kalzinieren des Produktes hergestellt werden. Bei Verwendung von vorgeformten Trägern können die einzelnen Metallkomponenten entweder zusammen oder aber auch hintereinander auf den Träger gebracht und in die aktive Form umgewandelt werden.

Die Konzentration des Isobutanals im Eingangsgemisch beträgt vorzugsweise 1 bis 4,5 Volumenprozent. Es sind aber auch höhere und niedrigere Konzentrationen möglich, jedoch sind die kleinen Konzentrationen wirtschaftlich uninteressant, und bei den höheren Konzentrationen wird, wenn man akzeptable Raum-Zeit-Ausbeuten voraussetzt, das Problem der Wärmeabfuhr schwierig.

Die Sauerstoff-Konzentration in dem Eingangsgas richtet sich nach der Isobutanal-Konzentration. Sie beträgt 1,2 bis 99 Volumenprozent im Eingangsgasgemisch. Das kleinste molare Verhältnis zwischen Isobutanal und Sauerstoff sollte 1:1,2 betragen. Bevorzugt werden molare Verhältnisse von Isobutanal zu Sauerstoff von 1:2 bis 1:3. Das Isobutanal-Sauerstoff-Verhältnis kann aber auch über diese Werte hinausgehen. So lässt sich die Reaktion auch mit reinem Sauerstoff durchführen, wenn die Isobutanal-Konzentration nicht zu hoch gewählt wird (ca. 1 bis 2,5 Volumenprozent). In der Regel wird dem Eingangsgas ein inertes Verdünnungsmittel zugegeben. Als Verdünnungsmittel kommen in Frage: Stickstoff, Wasserdampf, Kohlendioxid und Kohlenmonoxid. Bevorzugte Verdünnungsmittel sind Stickstoff und/oder Wasserdampf, wobei der Stickstoff aus der als bevorzugtes Oxidationsmittel verwendeten Luft stammt. Bei der Verwendung von reinem Wasserdampf bietet sich eine Fahrweise mit Sauerstoff als Kreisgas an. Die notwendigen Mengen an Verdünnungsmittel ergeben sich zwangsläufig aus den verwendeten Isobutanal- und Sauerstoff-Konzentrationen.

Die Reaktionstemperatur beträgt 150 bis 250°C. Obwohl auch unterhalb dieses Temperaturbereichs noch eine merkliche Reaktion stattfindet, ist doch die Reaktionsgeschwindigkeit unterhalb von 150°C für ein technisches Verfahren zu gering. Oberhalb 250°C wird die Ausbeute an Butan-2.3-dion merklich geringer. Besonders günstige Ergebnisse werden im Temperaturbereich von 180 bis 230°C erreicht.

Die Verweilzeit der Eingangsstoffe in dem Katalysatorbett (Kontaktzeit) richtet sich nach der Zusammensetzung des Eingangsgemisches und der Reaktionstemperatur und kann innerhalb eines Bereiches von 0,5 bis 8 sec schwanken. Sie liegt vorzugsweise in einem Bereich von 1 bis 4 sec. Die Umsetzung wird bei Normaldruck oder leicht erhöhten Drücken bis zu 5 bar durchgeführt. Der Druck wird nach oben hin durch die beginnende Kondensatbildung, das heisst Auftreten einer flüssigen Phase, begrenzt.

Als Koppelprodukt entsteht bei der Umsetzung Aceton, das mengenmässig sogar die Hauptmenge darstellt. Wertmässig ist jedoch das Butan-2.3-dion das Hauptprodukt. Daneben werden noch geringe Mengen (weniger als 1 Molprozent) an Isobutanol und β-Hydroxyisobutanal gefunden. Im Abgas befinden sich Kohlenmonoxid und Kohlendioxid. Das Abgas kann vorteilhaft noch als Heizgas eingesetzt werden.

Beispiel 1
480 g Zinkoxid werden zusammen mit 20 g Kolloidgraphit und 15 g Wasser innig gemischt und zu Tabletten (Durchmesser 4 mm, Dicke 4 mm) gepresst, anschliessend 16 Stunden bei 110°C getrocknet und 16 Stunden bei 350°C getempert. Die fertigen Tabletten werden mit 180 cm³ einer 13, 5prozentigen wässrigen Lösung von Mangannitrat imprägniert, 16 Stunden bei 110°C getrocknet und 16 Stunden bei 350°C kalziniert. Der fertige Katalysator enthält 1,5 Gewichtsprozent Mangan und 75,6 Gewichtsprozent Zink in Form ihrer Oxide und 3,9 Gewichtsprozent Graphit.

60 cm³ von dem so hergestellten Katalysator gibt man in ein mit siedendem Wasser thermostatisiertes Reaktionsrohr aus Stahl mit einem inneren Durchmesser von 20 mm und beaufschlagt ihn bei einer Reaktionstemperatur von 198°C, einer Kontaktzeit von 2 sec und einem Reaktorinnendruck von 1,5 bar mit einem Gasgemisch, das aus 3,5 Volumenprozent Isobutanal, 45,5 Volumenprozent Luft und 51,0 Volumenprozent Wasserdampf besteht. Das den Reaktor verlassende Gasgemisch untersucht man gaschromatographisch. Der Isobutanal-Umsatz beträgt 87%, und die Ausbeute an Butan-2.3-dion, bezogen auf die umgesetzte Menge an Isobutanal (Selektivität), beträgt 11,2 Molprozent und die Selektivität für Aceton 82,3 Molprozent, so dass die Gesamtselektivität für Butan-2.3-dion und Aceton 93,5 Molprozent beträgt.

Beispiel 2
64,6 g handelsübliches Titandioxid, 14,8 g Mangannitrat und 25 cm³ Wasser mischt man innig. Die erhaltene Suspension dampft man ein und kalziniert den Rückstand 16 Stunden bei 300°C. Das Produkt mahlt man anschliessend zusammen mit 2 g Kolloidgraphit, presst zu Tabletten (Durchmesser 4mm, Dicke 4 mm) und tempert 16 Stunden bei 350°C.

Den so hergestellten Katalysator setzt man unter den in Beispiel 1 beschriebenen Bedingungen bei einer Reaktionstemperatur von 236°C ein. Der Isobutanal-Umsatz beträgt 89,3%, und die Selektivitäten für Butan-2.3-dion und Aceton betragen 10,5 und 82,5 Molprozent, so dass die Gesamtselektivität für Butan-2.3-dion und Aceton 93 Molprozent beträgt.

## Patentanspruch

Verfahren zur Herstellung von Butan-2.3-dion und Aceton durch katalytische Oxidation von Isobutanal in der Gasphase, dadurch gekennzeichnet, dass man ein Gemisch aus 1 bis 4,5 Vol.-% Isobutanal, 1,2 bis 99 Vol.-% Sauerstoff und gegebenenfalls einem inerten Verdünnungsmittel bei Temperaturen von 150 bis 250°C, Reaktionsdrükken von 1 bis 5 bar und Kontaktzeiten von 0,5 bis 8 sec über einen Katalysator leitet, der aus Manganoxid und mindestens einem der Oxide von Zink oder Titan besteht, wobei in dem fertigen Katalysator die Manganoxidkonzentration 0,1 bis 10 Gewichtsprozent beträgt.

## Claim

A process for the manufacture of butane-2,3 dione and acetone by catalytic oxidation of isobutanal in the gas phase, characterized in that a mixture containing 1 – 4.5% by volume of isobutanal, 1.2 – 99% by volume of oxygen and, if necessary, an inert diluent is passed over a catalyst at a temperature of 150 – 250°C and a reaction pressure of 1 – 5 bar for a contact period of 0.5 – 8 sec., the catalyst consisting of manganese oxide and at least one of the oxides of zinc or titanium, the manganese oxide concentration in the finished catalyst amounting to 0.1 – 10% by weight.

## Revendication

Procédé pour la préparation de butane dione-2,3 et d'acétone par oxydation catalytique d'isobutanal dans la phase gazeuse, caractérisé par le fait qu'on introduit sur un catalyseur un mélange composé de 1 à 4,5% en volume d'isobutanal, 1,2 à 99% en volume d'oxygène et le cas échéant d'un diluant inerte, à des températures comprises entre 150 et 250°C, à des pressions de réaction de 1 à 5 bar et avec des durées de contact de 0,5 à 8 secondes, le catalyseur étant constitué d'oxyde de manganèse et au moins d'un des oxydes de zinc ou de titane, la concentration d'oxyde de manganèse dans le catalyseur final s'élevant entre 0,1 et 10% en poids.